(19) Europäisches Patentamt
European Patent Office
Office européen des brevets

(11) EP 1 428 843 B1

(12) FASCICULE DE BREVET EUROPEEN

(45) Date de publication et mention de la délivrance du brevet:
**18.04.2007 Bulletin 2007/16**

(51) Int Cl.:
***C08F 265/00*** *(2006.01)*

(21) Numéro de dépôt: **03293119.8**

(22) Date de dépôt: **12.12.2003**

# (54) Dispersions de polymères en milieu siliconé et composition les comprenant

Polymerdispersionen im Silikon Medium und deren Verwendung

Polymer dispersions in silicon medium and composition containing them

(84) Etats contractants désignés:
**AT BE BG CH CY CZ DE DK EE ES FI FR GB GR HU IE IT LI LU MC NL PT RO SE SI SK TR**

(30) Priorité: **12.12.2002 FR 0215739**

(43) Date de publication de la demande:
**16.06.2004 Bulletin 2004/25**

(73) Titulaire: **L'ORÉAL**
**75008 Paris (FR)**

(72) Inventeur: **Lion, Bertrand**
**95270 Luzarches (FR)**

(74) Mandataire: **Dodin, Catherine**
**L'OREAL - D.I.P.I.**
**25-29 Quai Aulagnier**
**92600 Asnières (FR)**

(56) Documents cités:
**EP-A- 0 210 041**
**EP-A- 0 250 093**
**EP-A- 0 412 704**
**EP-A- 0 412 707**
**WO-A-93/23446**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 428 843 B1

## Description

La présente invention a trait à des dispersions stables de particules formées par des polymères acryliques dans un milieu siliconé, ainsi qu'à l'utilisation de ces dispersions dans des compositions cosmétiques et aux compositions ainsi obtenues.

Il est connu d'utiliser dans le domaine de la cosmétique des dispersions de particules de polymère dans des milieux organiques, en tant qu'agents filmogènes dans différentes formulations de cosmétique, telles que les mascaras, les eye-liners, les ombres à paupières ou les vernis à ongles.

Ainsi, dans la demande de brevet européen EP-A-0749747, il est décrit une composition comprenant une dispersion de particules de polymères non solubles dans un milieu non aqueux, ladite dispersion étant stabilisée par ajout de polymères stabilisants. Les polymères stabilisants selon ce document se lient de manière non covalente par le biais d'interactions physiques sur les polymères non solubles évoqués ci-dessus.

Toutefois, ce type de composition présente les inconvénients suivants : elle nécessite l'ajout dans le milieu non aqueux d'une quantité de polymères dits stabilisants supérieure à celle effectivement liée aux particules de polymères non solubles, afin d'obtenir une dispersion desdites particules relativement stable. Or, lors de l'ajout dans ces compositions d'adjuvants, tels que des pigments, une partie des polymères stabilisants a tendance à se désorber des particules de polymères non solubles pour s'associer avec lesdits adjuvants, ce qui contribue à déstabiliser la dispersion, notamment par formation d'agglomérats entre les particules de polymères.

Le document JP 11181003 décrit des polymères aptes à former des particules solides sans ajout de polymères stabilisants mais toutefois ces particules sont instables dans des milieux organiques non aqueux.

La demanderesse a découvert de manière surprenante de nouveaux polymères, aptes à former des particules stables dans un milieu siliconé, sans ajout de polymères stabilisants.

Ainsi, la présente invention a pour but de proposer une dispersion, dans un milieu organique siliconé, de particules individuelles autostabilisées de polymères, ladite dispersion étant exempte d'agglomérats de particules et de sédiments insolubles, visuellement, par exemple après mise au repos de la dispersion une journée (24 heures) à température ambiante (20°C).

Par ailleurs, on a constaté que certaines des dispersions de particules de polymères dans un milieu non aqueux, préparées dans l'art antérieure, présentaient une mauvaise tenue au sébum, ou au gras (par exemple à l'huile). Ceci peut être rédhibitoire pour certaines catégories de compositions cosmétiques, en particulier pour les compositions de maquillage telles que les fonds de teint ou les rouges à lèvres, qui présentent une mauvaise tenue dans le temps.

La présente invention a donc également pour but de proposer une composition ayant une tenue améliorée, notamment au gras et au sébum, ainsi que de bonnes propriétés de non-transfert, tout en ne présentant pas de problème de collant.

Un premier objet de la présente invention est donc une dispersion, dans un milieu non aqueux siliconé de particules notamment solides constituées d'au moins un polymère acrylique comprenant un squelette insoluble dans ledit milieu, et une partie soluble dans ledit milieu constituée de chaînes latérales liées de manière covalente audit squelette, ledit polymère étant susceptible d'être obtenu par polymérisation radicalaire dans ledit milieu d'un mélange polymérisable constitué :

- d'un premier monomère choisi parmi les (méth)acrylates d'alkyle en C1-C3, seul ou en mélange, éventuellement en présence d'un ou plusieurs monomères additionnels choisis parmi l'acide acrylique, l'acide méthacrylique et les (méth)acrylates d'alkyle de formule (I), et leurs sels, pour former ledit squelette insoluble; et
- d'au moins un macromonomère siliconé comportant un groupe terminal apte à réagir pendant la polymérisation pour former les chaînes latérales, ledit macromonomère ayant une masse moléculaire en poids supérieure ou égale à 200 et représentant 1 à 18% en poids du polymère.

Un autre objet de l'invention est une composition cosmétique ou pharmaceutique comprenant, dans un milieu cosmétiquement ou pharmaceutiquement acceptable, une dispersion telle que ci-dessus définie.

Les dispersions selon l'invention sont donc exemptes de polymère stabilisant, tels que ceux décrits dans EP749747, et les polymères selon l'invention ne sont donc pas stabilisés en surface par de tels polymères stabilisants additionnels.

La dispersion selon l'invention comprend donc un milieu siliconé non aqueux.

On entend par "milieu non aqueux", un milieu constitué d'un ou plusieurs composés siliconés tels que définis ci-après, ledit milieu pouvant comprendre au plus 1 % en poids d'eau.

On entend par "milieu siliconé" un milieu constitué majoritairement d'un ou plusieurs composés siliconés tels que définis ci-après, c'est-à-dire un milieu dans lequel lesdits composés siliconés représentent au moins 50% en poids, notamment de 50 à 100% en poids, par exemple de 60 à 99% ou encore de 65 à 95% en poids, de l'ensemble des constituants liquides 'composés siliconés + composés non siliconés éventuels + eau éventuelle' constituant ledit milieu.

Ledit milieu peut donc éventuellement comprendre des composés non siliconés qui peuvent être présents en une quantité maximale de 50% en poids, notamment de 0 à 40% en poids, voire de 1 à 35% en poids, et encore de 5-30% en poids par rapport au poids total du milieu.
De préférence, le milieu siliconé non aqueux est liquide.

Parmi les composés siliconés susceptibles d'être présents dans ledit milieu siliconé, on peut citer les composés liquides non aqueux siliconés ayant un paramètre de solubilité global selon l'espace de solubilité de Hansen inférieur ou égal à 17 $(MPa)^{1/2}$.

Le paramètre de solubilité global δ selon l'espace de solubilité de Hansen est défini dans l'article « Solubility parameter values » de Eric A.Grulke de l'ouvrage « Polymer Handbook », 3ème édition, Chapitre VII, p.519-559 par la relation :

$$\delta = (d_D^2 + d_P^2 + d_H^2)^{1/2}$$

dans laquelle

- $d_D$ caractérise les forces de dispersion de LONDON issues de la formation de dipôles induits lors des chocs moléculaires,
- $d_P$ caractérise les forces d'intéractions de DEBYE entre dipôles permanents, et
- $d_H$ caractérise les forces d'intéractions spécifiques (type liaisons hydrogène, acide/base, donneur/accepteur, etc.).

La définition des solvants dans l'espace de solubilité selon Hansen est décrite dans l'article de C.M.Hansen « The three dimensional solubility parameters » J.Paint Technol. 39, 105 (1967).

En particulier, on peut citer les huiles de silicone, éventuellement ramifiées, volatiles et/ou non volatiles.
Par huile volatile, on entend une huile susceptible de s'évaporer de la peau ou des lèvres en moins d'une heure, ayant notamment une pression de vapeur, à température ambiante et pression atmosphérique allant de $10^{-3}$ à 300 mm de Hg (0,13 Pa à 40 000 Pa).
Comme huile siliconée volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone. En particulier, on peut citer l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.
Comme huile siliconée non volatile, on peut citer les polydialkylsiloxanes non volatils, tels que les polydiméthylsiloxanes (PDMS) non volatils; les polydiméthylsiloxanes comportant des groupements alkyle, alcoxy ou phényle, pendant ou en bout de chaîne siliconée, groupements ayant de 2 à 24 atomes de carbone; les silicones phénylées comme les phényl triméthicones, les phényl diméthicones, les phényl triméthylsiloxy diphénylsiloxanes, les diphényl diméthicones, les diphényl méthyldiphényl trisiloxanes, les polyméthylphénylsiloxanes; les polysiloxanes modifiés par des acides gras (notamment en $C_8$-$C_{20}$), des alcools gras (notamment en $C_8$-$C_{20}$) ou des polyoxyalkylènes (notamment polyoxyéthyléne et/ou polyoxypropylène); les polysiloxanes aminées ; les polysiloxanes à groupement hydroxyles; les polysiloxanes fluorés comportant un groupement fluoré pendant ou en bout de chaîne siliconée ayant de 1 à 12 atomes de carbone dont tout ou partie des hydrogène sont substitués par des atomes de fluor ; et leurs mélanges.

Parmi les composés non siliconés éventuellement présents, en faible quantité, dans le milieu siliconé, on peut citer les composés liquides non aqueux non siliconés ayant un paramètre de solubilité global selon l'espace de solubilité de Hansen inférieur ou égal à 18 $(MPa)^{1/2}$, de préférence inférieur ou égal à 17 $(MPa)^{1/2}$; les monoalcools ayant un paramètre de solubilité global selon l'espace de solubilité de Hansen inférieur ou égal à 20 $(MPa)^{1/2}$; et leurs mélanges.
Parmi les composés liquides non aqueux non siliconés, on peut citer des corps gras liquides, notamment des huiles, qui peuvent être choisies parmi les huiles naturelles ou synthétiques, carbonées, hydrocarbonées, fluorées, éventuellement ramifiées, seules ou en mélange. Parmi ces huiles, on peut citer les huiles végétales formées par des esters d'acides gras et de polyols, en particulier les triglycérides, telles que l'huile de tournesol, de sésame ou de colza, ou les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates et les benzoates, notamment l'adipate de diisopropyle. On peut également citer les alcanes linéaires, ramifiés et/ou cycliques éventuellement volatils et notamment des huiles de paraffine, de vaseline, ou le polyisobutylène hydrogéné, l'isododécane, ou encore les 'ISOPARS', les isoparaffines volatiles. On peut citer également les esters linéaires, ramifiés ou cycliques, ayant plus de 6 atomes de carbone ; les éthers ayant plus de 6 atomes de carbone ; et les cétones ayant plus de 6 atomes de carbone.

Par monoalcools ayant un paramètre de solubilité global selon l'espace de solubilité de Hansen inférieur ou égal à 20 $(MPa)^{1/2}$, on entend les monoalcools gras aliphatiques ayant 6 à 30 atomes de carbone, la chaîne hydrocarbonée ne comportant pas de groupement de substitution, parmi lesquels l'alcool oléique, le décanol, le dodécanol, l'octadécanol et l'alcool linoléique.

Le choix des monomères constituant le squelette des polymères et des macromonomères, de même que la masse moléculaire du polymère, des chaînes latérales ainsi que la proportion des monomères et des macromonomères dans le polymère se fera en fonction du milieu siliconé de manière à obtenir une dispersion de particules solides de polymères stable dans ledit milieu, ce choix pouvant être effectué par l'homme du métier.

Par "dispersion stable", on entend, selon l'invention, une dispersion qui n'est pas susceptible de former de dépôt solide ou de déphasage liquide/solide notamment après une centrifugation, par exemple, à 4000 tours/minute pendant 15 minutes.

Les polymères acryliques formant les particules en dispersion comprennent donc un squelette insoluble dans ledit milieu et une partie soluble dans ledit milieu.

Ces polymères peuvent se présenter sous différentes formes, en particulier sous forme de polymères statistiques.

Selon l'invention, on entend par "polymère acrylique" un polymère susceptible d'être obtenu par polymérisation radicalaire d'un mélange polymérisable constitué :

- de premier monomère de type (méth)acrylate d'alkyle en C1-C3, seul ou en mélange,
- d'éventuels monomères additionnels choisis parmi l'acide acrylique, l'acide méthacrylique, les (méth)acrylates d'alkyle de formule (I), et leurs sels
- avec un ou plusieurs macromonomères siliconés, dans un milieu siliconé non aqueux donné ou dans un milieu de polymérisation.

De préférence, le premier monomère, ou mélange de premiers monomères, représente 50-100% en poids, notamment 55 à 95% en poids, voire 60 à 80% en poids du mélange "premier(s) monomère(s) + monomère(s) additionnel (s)".

Selon l'invention, on entend par "macromonomère" tout polymère, notamment oligomère, comportant sur une seule de ses extrémités un groupe terminal, notamment polymérisable, apte à réagir lors de la réaction de polymérisation avec les monomères considérés, pour former les chaînes latérales, ledit groupe pouvant être avantageusement un groupe à insaturation éthylénique susceptible de se polymériser par voie radicalaire avec les monomères constituant le squelette.

De préférence, le macromonomère est choisi parmi les macromonomères dont l'homopolymère est soluble dans le milieu siliconé considéré, c'est-à-dire complètement dissous à une concentration supérieure ou égale à 5% en poids et à température ambiante dans ledit milieu.

Ainsi, les polymères selon l'invention se présentent sous la forme de polymères insolubles dans le milieu considéré, et comprennent un squelette (ou chaîne principale) constitué par un enchaînement de motifs notamment acryliques résultant de la polymérisation notamment d'un ou plusieurs monomères acryliques et des chaînes latérales (ou greffons) issus de la réaction des macromonomères siliconés, lesdites chaînes latérales étant liées de manière covalente à ladite chaîne principale.

Le squelette (ou chaîne principale) est insoluble dans le milieu considéré alors que les chaînes latérales (ou greffons) sont solubles dans ledit milieu.

Comme premier monomère susceptible d'être employé pour constituer après polymérisation le squelette insoluble du polymère, on peut citer, seul ou en mélange, l'acrylate de méthyle, le méthacrylate de méthyle, l'acrylate d'éthyle, le méthacrylate d'éthyle, l'acrylate de n-propyle, le méthacrylate de n-propyle, l'acrylate d'iso-propyle et le méthacrylate d'isopropyle.

On préfère tout particulièrement l'acrylate de méthyle, le méthacrylate de méthyle et d'éthyle.

Les monomères additionnels, éventuellement employés avec ledit premier monomère ou mélange de premiers monomères, pour constituer après polymérisation le squelette insoluble du polymère acrylique, sont choisis parmi, seul ou en mélange, :

- l'acide (méth)acrylique et ses sels,
- les (méth)acrylates de formule (I) et leurs sels :

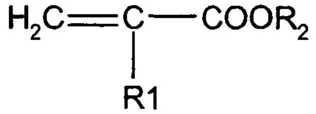

(I)

dans laquelle :

- $R_1$ désigne un atome d'hydrogène ou un groupe méthyle ;
- $R_2$ représente
  - un groupe alkyle linéaire ou ramifié comprenant de 1 à 6 atomes de carbone, ledit groupe comportant dans sa chaîne un ou plusieurs atomes d'oxygène, et/ou comportant un ou plusieurs substituants choisis parmi - OH, les atomes d'halogène (F, Cl, Br, I) et -NR'R" avec R' et R" identiques ou différents choisis parmi les alkyles linéaires ou ramifiés en C1-C3;
  - un groupe alkyle cyclique comprenant de 3 à 6 atomes de carbone, ledit groupe pouvant comporter dans sa chaîne un ou plusieurs atomes d'oxygène et/ou pouvant comporter un ou plusieurs substituants choisis parmi OH et les atomes d'halogène (F, Cl, Br, I).

A titre d'exemples de $R_2$, on peut citer le groupe méthoxyéthyle, éthoxyéthyle, trifluoroéthyle; 2-hydroxyéthyle, 2-hydroxypropyle, diméthylaminoéthyle, diéthylaminoéthyle, diméthylaminopropyle.

Parmi ces monomères additionnels, on peut tout particulièrement citer, seuls ou en mélange, les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle et leurs sels.

On préfère tout particulièrement l'acide acrylique et l'acide méthacrylique.

Parmi les sels, on peut citer ceux obtenus par neutralisation des groupements acides à l'aide de base inorganiques telles que l'hydroxyde de sodium, l'hydroxyde de potassium, l'hydroxyde d'ammonium ou de bases organiques de type alkanols amines comme la monoéthanolamine, la diéthanolamine, la triéthanolamine, la 2-méthyl-2-amino-1-propanol.

On peut également citer ceux formés par neutralisation des motifs amine tertiaire, par exemple à l'aide d'acide minéral ou organique. Parmi les acides minéraux, on peut citer l'acide sulfurique ou l'acide chlorhydrique, l'acide bromhydrique, iodhydrique, l'acide phosphorique, l'acide borique. Parmi les acides organiques, on peut citer les acides comportant un ou plusieurs groupes carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles. On peut notamment citer l'acide acétique ou l'acide propionique, l'acide téréphtalique, ainsi que l'acide citrique et l'acide tartrique.

Il est entendu que ces monomères, premier et additionnels, non polymérisés peuvent être solubles dans le milieu considéré, mais deviennent insolubles après polymérisation en une quantité appropriée, ce qui est l'objectif de la présente invention.

Les macromonomères constituant après réaction les chaînes latérales du polymère selon l'invention, comportent en bout de chaîne un groupe terminal apte à réagir au cours de la polymérisation avec les monomères acryliques et vinyliques, pour former lesdites chaînes, ledit groupe terminal polymérisable étant en particulier un groupe vinyle ou de préférence un groupe (méth)acryloyloxy (acrylate ou méthacrylate).

Les macromonomères sont choisis préférentiellement parmi les macromonomères siliconés, en particulier ceux dont les homopolymères présentent une température de transition vitreuse (Tg) inférieure ou égale à 25°C, notamment allant de -100°C à 25°C, de préférence allant de -80°C à 0°C inclus.

De préférence, les macromonomères selon l'invention présentent une masse moléculaire moyenne en poids (Mw) allant de 200 à 100 000, de préférence de 300 à 50 000, notamment 500 à 20 000, préférentiellement de 800 à 10000, voire de 1000 à 6000.

On peut en particulier citer les polydiméthylsiloxanes à groupement terminal monoacryloxy ou monométhacryloxy, et notamment ceux de formule (II) suivante :

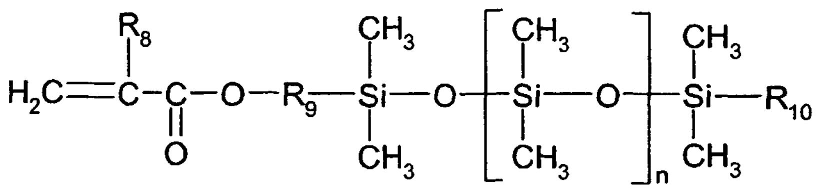 (II)

dans laquelle :

- $R_8$ désigne un atome d'hydrogène ou un groupement méthyle ; de préférence méthyle;
- $R_9$ désigne un groupe hydrocarboné divalent, linéaire ou ramifié, de préférence linéaire, ayant de 1 à 10 atomes de carbone et contenant éventuellement une ou deux liaisons éther -O- ; de préférence éthylène, propylène ou butylène;
- $R_{10}$ désigne un groupe alkyle linéaire ou ramifié, ayant de 1 à 10 atomes de carbone, notamment de 2 à 8 atomes de carbone; de préférence méthyle, éthyle, propyle, butyle ou pentyle;
- n désigne un nombre entier allant de 1 à 300, de préférence allant de 3 à 200, et préférentiellement allant de 5 à 100.

On peut en particulier utiliser les monométhacryloxypropyl polydiméthylsiloxanes tels que ceux commercialisés sous la dénomination PS560-K6 par UCT (United Chemical Technologies Inc.) ou sous la dénomination MCR-M17 par Gelest Inc.

Les macromonomères sont de préférence présents dans les polymères de l'invention dans une proportion de 1 à 18% en poids, par exemple 2 à 16% en poids, préférentiellement de 4 à 15% en poids, et notamment de 6 à 12%, voire de 8% à 10% en poids, par rapport au poids total dudit polymère.

Des polymères particulièrement avantageux selon l'invention sont ceux obtenus par polymérisation :

- d'acrylate de méthyle et d'un macromonomère monométhacryloxypropylpolydiméthylsiloxane ayant un Mw allant de 800 à 5000, en particulier dans le décaméthylcyclopentasiloxane ou le phényltriméthicone;
- d'acrylate de méthyle, d'acide acrylique et d'un macromonomère monométhacryloxypropylpolydiméthylsiloxane ayant un Mw allant de 800 à 5000, en particulier dans le décaméthylcyclopentasiloxane ou le phényltriméthicone.

De préférence, la masse moléculaire en poids (Mw) du polymère est comprise entre 10 000 et 300 000, notamment entre 20 000 et 200 000, mieux encore entre 25 000 et 150 000.

Grâce aux caractéristiques susmentionnées, dans un milieu siliconé donné, les polymères ont la capacité de se replier sur eux-mêmes, formant ainsi des particules de préférence solides de forme sensiblement sphérique, avec sur le pourtour de ces particules les chaînes latérales déployées, qui assurent la stabilité de ces particules. De telles particules résultant des caractéristiques des polymères de l'invention, ont la particularité de ne pas s'agglomérer dans ledit milieu et donc de s'autostabiliser et de former une dispersion de particules de polymère particulièrement stable.
En particulier, les polymères de l'invention sont aptes à former des particules solides nanométriques, de taille moyenne allant de 10 à 400 nm, de préférence de 20 à 200 nm..
Du fait de cette taille très faible, les particules solides entrant dans la constitution de la dispersion sont particulièrement stables et donc peu susceptibles de former des agglomérats.
La dispersion de l'invention est donc une dispersion stable dans le milieu considéré et ne forme pas de sédiments, lorsqu'elle est placée pendant une durée prolongée (par exemple 24 heures) à température ambiante (25°C).

De préférence, la dispersion de particules présente un taux de matière sèche (ou extrait sec) de 40% à 70% en poids de matière sèche, notamment de 45 à 65% en poids.

On peut préparer ledit polymère ou ladite dispersion de particules de polymère, par un procédé comprenant une étape consistant à réaliser une copolymérisation radicalaire, dans un milieu répondant à la définition donnée précédemment, d'un ou plusieurs monomères acryliques tels que définis précédemment avec un ou plusieurs macromonomères siliconés tels que définis précédemment.
D'une manière classique, la copolymérisation peut être effectuée en présence d'un initiateur de polymérisation. Les initiateurs de polymérisation peuvent être des amorceurs radicalaires. De manière générale, un tel initiateur de polymérisation peut être choisi parmi les composés organiques peroxydés tels que le dilauroyl peroxyde, le dibenzoyl peroxyde, le tert-butyl peroxy-2-éthylhexanoate ; les composés diazotés tels que l'azobisisobutyronitrile, l'azobisdiméthylvaleronitrile.
La réaction peut être également initiée à l'aide de photoinitiateurs ou par une radiation telle que des UV, des neutrons ou par plasma.
D'une manière générale, pour mettre en oeuvre ce procédé, on introduit, dans un réacteur de taille appropriée à la quantité de polymère que l'on va réaliser, au moins une partie du milieu siliconé, une partie des monomères acryliques et/ou additionnels, qui constituera, après polymérisation, le squelette insoluble, la totalité du macromonomère (qui constituera les chaînes latérales du polymère) et une partie de l'initiateur de polymérisation. A ce stade d'introduction, le milieu réactionnel forme un milieu relativement homogène.
Le milieu réactionnel est ensuite agité et chauffé jusqu'à une température pour obtenir une polymérisation des monomères et macromonomères. Après un certain temps, le milieu initialement homogène et limpide conduit à une dispersion d'aspect laiteux. On ajoute ensuite un mélange constitué de la partie restante de monomère et de l'initiateur de polymérisation. Après un temps adéquat pendant lequel le mélange est chauffé sous agitation, le milieu se stabilise sous forme d'une dispersion laiteuse, la dispersion comprenant des particules de polymères stabilisés dans le milieu dans lequel elles ont été créées, ladite stabilisation étant due à la présence de chaînes latérales solubles dans ledit milieu.

Il est également possible de préparer la dispersion de particules solides de polymère dans un milieu de polymérisation qui peut ultérieurement être remplacé par un milieu siliconé non aqueux au sens de l'invention.

La dispersion de particules solides de polymères selon l'invention peut être utilisée dans tout type de composition et notamment dans une composition cosmétique ou pharmaceutique comprenant un milieu cosmétiquement ou pharmaceutiquement acceptable, telle qu'une composition de soin, de nettoyage ou de maquillage pour la peau ou des matières kératiniques, ou une composition capillaire ou une composition solaire.

La dispersion peut être présente à raison de 3 à 95% en poids dans la composition, notamment 4-90% en poids, voire, 20-70% en poids.

De préférence, la composition comprend 0,5 à 25% en poids de matière active de polymère selon l'invention, notamment 1 % à 20% en poids, en particulier 4% à 17% en poids, par exemple 5% à 15% en poids, par rapport au poids total de la composition.

Selon l'application recherchée, la composition peut contenir les adjuvants habituels que l'on incorpore dans des compositions cosmétiques ou pharmaceutiques.

Parmi ces adjuvants, on peut citer les corps gras, et notamment les cires, les huiles, les gommes et/ou les corps gras pâteux, hydrocarbonés et/ou siliconés, et les composés pulvérulents tels que les pigments, les charges et/ou les nacres.

Parmi les cires susceptibles d'être présentes dans la composition selon l'invention, on peut citer, seules ou en mélange, les cires hydrocarbonées telles que la cire d'abeilles ; la cire de Carnauba, de Candellila, d'Ourrury, du Japon, les cires de fibres de liège ou de canne à sucre ; les cires de paraffine, de lignite ; les cires microcristallines ; la cire de lanoline ; la cire de montan ; les ozokérites ; les cires de polyéthylène ; les cires obtenues par synthèse de Fischer-Tropsch ; les huiles hydrogénés, les esters gras et les glycérides concrets à 25°C. On peut également utiliser des cires de silicone, parmi lesquelles on peut citer les alkyls, alcoxys, et/ou esters de polyméthylsiloxane.

Parmi les huiles susceptibles d'être présentes dans la composition selon l'invention, on peut citer, seules ou en mélange, les huiles hydrocarbonées telles que l'huile de paraffine ou de vaseline; le perhydrosqualène ; l'huile d'acara ; l'huile d'amande douce, de calophyllum, de palme, de ricin, d'avocat, de jojoba, d'olive ou de germes de céréales; des esters d'acide lanolique, d'acide oléique, d'acide laurique, d'acide stéarique ; des alcools tels que l'alcool oléique, l'alcool linoléique ou linolénique, l'alcool isostéarique ou l'octyl dodécanol. On peut également citer les huiles siliconées telles que le PDMS, éventuellement phénylées telles que les phényltriméthicones. On peut également citer des huiles volatiles, telles que la cyclotétradiméthylsiloxane, la cyclopentadiméthylsiloxane, la cyclohexadiméthylsiloxane, le méthylhexyldiméthylsiloxane, l'hexaméthyldisiloxane ou les isoparaffines.

Les pigments peuvent être blancs ou colorés, minéraux et/ou organiques. On peut citer parmi les pigments minéraux, les dioxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium.

Les nacres peuvent être choisies parmi le mica recouvert d'oxyde de titane, d'oxyde de fer, de pigment naturel ou d'oxychlorure de bismuth ainsi que le mica titane coloré.

Les charges peuvent être minérales ou de synthèse, lamellaires ou non lamellaires. On peut citer le talc, le mica, la silice, le kaolin, les poudres de nylon et de polyéthylène, le Téflon, l'amidon, le micatitane, le nacre naturelle, le nitrure de bore, les microsphères creuses telles que l'Expancel (Nobel Industrie), le polytrap (Dow Corning) et les microbilles de résine de silicone (Tospearls de Toshiba, par exemple).

La composition peut comprendre en outre tout additif usuellement utilisé dans le domaine cosmétique, tel que des antioxydants, des parfums, des huiles essentielles, des conservateurs, des actifs cosmétiques, des hydratants, des vitamines, des acides gras essentiels, des sphingolipides, des filtres solaires, des tensioactifs, des polymères liposolubles comme les polyalkylènes, notamment le polybutène, les polyacrylates et les polymères siliconés compatibles avec les corps gras. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas altérées par l'adjonction envisagée.

Les compositions selon l'invention peuvent se présenter sous toute forme acceptable et usuelle pour une composition cosmétique, hygiénique ou pharmaceutique, et notamment sous forme d'émulsion huile-dans-eau ou eau-dans huile, de lotion, de mousse, de spray.

Parmi les applications préférentiellement visées par la présente invention, on peut plus particulièrement mentionner :

- le domaine des produits capillaires (lavage, soin ou beauté des cheveux), les compositions selon l'invention étant en particulier sous forme d'aérosols, de mousse, de shampooings, d'après-shampooings, de lotions ou de gels coiffants ou traitants, laques ou lotions de mise en forme ou de mise en plis ou encore de fixation ;
- le domaine des produits de maquillage, en particulier pour le maquillage des cils, les compositions étant sous forme de mascara ou de eye-liner ; de rouge à lèvres ou de brillant à lèvres ou de fond de teint, ou encore de fards à joues ou à paupières ;

- le domaine des produits de soin de la peau do corps et du visage, notamment les produits solaires ou autobronzants.

La présente invention a également pour objet un procédé de traitement cosmétique pour le soin, le nettoyage et/ou le maquillage des matières kératiniques telles que la peau, le cuir chevelu, les cils, les sourcils, les lèvres, les ongles, consistant à appliquer sur lesdites matières kératiniques, une composition telle que définie précédemment.

L'invention va maintenant être décrite plus en détail à la lumière des exemples suivants donnés à titre illustratif et non limitatif.

Les présents exemples illustrent la préparation de polymères conformes à l'invention, aptes à former une dispersion de particules dans le milieu considéré.

Dans ces exemples, on détermine, après préparation de ladite dispersion, les masses molaires moyennes en poids (Mw) et en nombre (Mn) du polymère, la température de transition vitreuse du polymère, le taux de matière sèche (ou extrait sec) de la dispersion et la taille des particules de polymères.

Les masses molaires moyennes en poids (Mw) et en nombre (Mn) sont déterminées par chromatographie liquide par perméation de gel (solvant THF, courbe d'étalonnage établie avec des étalons de polystyrène linéaire, détecteur réfractométrique).

La mesure de la température de transition vitreuse (Tg) est effectuée selon la norme ASTM D3418-97, par analyse enthalpique différentielle (DSC "Differential Scanning Calorimetry") sur calorimètre, sur une plage de température comprise entre -100°C et +150°C à une vitesse de chauffe de 10°C/min dans des creusets en aluminium de 150 μl.

La préparation des creusets se fait de la manière suivante : dans un creuset en aluminium de 150 μl on introduit 100 μl de la dispersion obtenue et on laisse le solvant s'évaporer pendant 24h à température ambiante et à 50% d'HR. On renouvelle l'opération puis on introduit le creuset dans le calorimètre Mettler DSC30.

Le taux de matière sèche (ou extrait sec), c'est à dire la teneur en matière non volatile, peut être mesurée de différentes manières : on peut citer par exemple les méthodes par séchage à l'étuve ou les méthodes par séchage par exposition à un rayonnement infrarouge.

De préférence, le taux de matière sèche est mesuré par échauffement de l'échantillon par des rayons infrarouges de 2 μm à 3,5 μm de longueur d'onde. Les substances contenues dans la composition qui possèdent une pression de vapeur élevée s'évaporent sous l'effet de ce rayonnement. La mesure de la perte de poids de l'échantillon permet de déterminer l'extrait sec de la composition. Ces mesures sont réalisées au moyen d'un dessiccateur à infrarouges commercial LP16 de chez Mettler. Cette technique est parfaitement décrite dans la documentation de l'appareil fournie par Mettler.

Le protocole de mesure est le suivant : on étale environ 1g de la composition sur une coupelle métallique. Celle-ci, après introduction dans le dessiccateur, est soumise à une consigne de température de 120°C pendant une heure. La masse humide de l'échantillon, correspondant à la masse initiale et la masse sèche de l'échantillon, correspondant à la masse après exposition au rayonnement, sont mesurées au moyen d'une balance de précision.

Le taux de matière sèche est calculée de la manière suivante :

$$\text{Extrait Sec} = 100 \times (\text{masse sèche / masse humide}).$$

Les tailles de particules peuvent être mesurées par différentes techniques : on peut citer en particulier les techniques de diffusion de la lumière (dynamiques et statiques), les méthodes par compteur Coulter, les mesures par vitesse de sédimentation (reliée à la taille via la loi de Stokes) et la microscopie. Ces techniques permettent de mesurer un diamètre de particules et pour certaines d'entre elles une distribution granulométrique.

De préférence, les tailles et les distributions de tailles des particules des compositions selon l'invention, sont mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957.

La composition est caractérisée par son diamètre "effectif" moyen en volume D[4,3], défini de la manière suivante :

$$D[4,3] = \frac{\sum_i V_i \cdot d_i}{\sum_i V_i}$$

où $V_i$ représente le volume des particules de diamètre effectif $d_i$. Ce paramètre est notamment décrit dans la documentation technique du granulomètre.

Les mesures sont réalisées à 25°C, sur une dispersion de particules diluée, obtenue à partir de la composition de la manière suivante : 1) dilution d'un facteur 100 avec de l'eau, 2) homogénéisation de la solution, 3) repos de la solution durant 18 heures, 4) récupération du surnageant homogène blanchâtre.

Le diamètre « effectif » est obtenu en prenant un indice de réfraction de 1,33 pour l'eau et un indice de réfraction moyen de 1,42 pour les particules.

## EXEMPLE 1

Cet exemple illustre la préparation d'un polymère formant une dispersion de particules dans un solvant siliconé, ledit polymère étant obtenu par polymérisation d'acrylate de méthyle en présence de macromonomère monométhacryloxypropylpolydiméthylsiloxane (PS560- K6)

Dans un réacteur de 500 ml, on charge 150 g de décaméthylcyclopentasiloxane, 12,75 g d'acrylate de méthyle, 2,25 g de monométhacryloxypropylpolydiméthylsiloxane (PS560-K6 de Mw = 1000) et 0,8 g de tertiobutyl peroxy-2-éthylhexanoate (Trigonox 21S).

On agite et on chauffe le mélange réactionnel à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 35 g d'acrylate de méthyle et 0,5 g de Trigonox 21S. On laisse ensuite le chauffage pendant 4 heures à 90°C A l'issue de cette opération de chauffage, on obtient une dispersion de particules de polymère stable dans le décaméthylcyclopentasiloxane (D5).

Les caractéristiques du polymère et des particules formées par ledit polymère sont les suivantes :

- Masse moléculaire poids Mw : 186800
- Masse moléculaire nombre Mn : 36830
- Indice de polydispersité (Mw/Mn)= 5,07
- Transition vitreuse : 7°C par DSC Mettler
- Extrait sec : 25% dans le D5, réalisé par thermobalance
- Granulométrie : 160 nm avec polydispersité de 0,05 réalisée sur Malvern Autosizer Lo-C à 25°C.

Le macromonomère représente 4,5% en poids du polymère.

La stabilité de la dispersion obtenue est mise en évidence par la mise en oeuvre du protocole de stabilité suivant : dans un tube à hémolyse, on place 8 ml de la dispersion réalisée et on centrifuge à 4000 tours/min pendant 15 minutes à l'aide d'une centrifugeuse Jouan C100-S5. Au bout de 15 minutes, on constate qu'il n'y a pas de déphasage ce qui démontre que la dispersion est stable.

## EXEMPLE 2

Cet exemple illustre la préparation d'un polymère formant une dispersion de particules dans un solvant siliconé, ledit polymère étant obtenu par polymérisation d'acrylate de méthyle en présence d'un macromonomère monométhacryloxypropylpolydiméthylsiloxane (MCR-M17).

Dans un réacteur de 1 litre, on charge 200 g d'heptane, 200 g de décaméthylcyclopentasiloxane, 30 g d'acrylate de méthyle, 10 g de macromonomère monométhacryloxypropylpolydiméthylsiloxane (MCR-M17 de Mw = 5000) et 3,2 g de tertiobutylperoxy-2-éthylhexanoate (Trigonox 21S).

On agite et on chauffe le mélange réactionnel à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 160 g d'acrylate de méthyle et 2 g de Trigonox 21S. On laisse ensuite le chauffage pendant 4 heures à 90°C puis on distille l'heptane du milieu réactionnel.

A l'issue de cette opération de distillation, on obtient une dispersion de particules de polymère ainsi préparé stable dans le décaméthylcyclopentasiloxane (D5).

Les caractéristiques du polymère et des particules formées par ledit polymère sont les suivantes :

- Masse moléculaire poids Mw=102347
- Masse moléculaire nombre Mn=28283
- Indice de polydispersité (Mw/Mn)= 3.62
- Extrait sec : 51,4 % dans le D5 réalisé par thermobalance

- Transition vitreuse : 12°C par DSC Mettler
- Granulométrie : 160 nm avec polydispersité de 0,04 réalisée sur Malvern Autosizer Lo-C à 25°C

Le macromonomère représente 5% en poids du polymère.
Après mise en oeuvre du protocole de stabilité conformément à l'exemple 1, on constate que la dispersion est stable.

**EXEMPLE 3**

Cet exemple illustre la préparation d'un polymère formant une dispersion de particules dans un solvant siliconé, ledit polymère étant obtenu par polymérisation d'acrylate de méthyle et de macromonomère monométhacryloxypropylpolydiméthylsiloxane (MCR-M17).
Dans un réacteur de 1 litre, on charge 200 g d'heptane, 200 g de décaméthylcyclopentasiloxane, 30 g d'acrylate de méthyle, 16 g de macromonomère monométhacryloxypropylpolydiméthylsiloxane (MCR-M17 de Mw = 5000) et 3,2 g de tertiobutylperoxy-2-éthylhexanoate (Trigonox 21S).
On agite et on chauffe le mélange réactionnel à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 154 g d'acrylate de méthyle et 2 g de Trigonox 21S. On laisse ensuite le chauffage pendant 4 heures à 90°C puis on distille l'heptane du milieu réactionnel.
A l'issue de cette opération de distillation, on obtient une dispersion de particules de polymère ainsi préparé stable dans le décaméthylcyclopentasiloxane
Les caractéristiques du polymère et des particules formées par ledit polymère sont les suivantes :

- Masse moléculaire poids Mw=118986
- Masse moléculaire nombre Mn=29914
- Indice de polydispersité (Mw/Mn)= 3.98
- Extrait sec : 49,6 % dans le D5 réalisé par thermobalance
- Transition vitreuse : 12°C par DSC Mettler
- Granulométrie : 170 nm avec polydispersité de 0,04 réalisée sur Malvern Autosizer Lo-C à 25°C

Le macromonomère représente 8% en poids du polymère.
Après la mise en oeuvre du protocole de stabilité conformément à l'exemple 1, on constate que la dispersion obtenue est stable.

**EXEMPLE 4**

Cet exemple illustre la préparation d'un polymère formant une dispersion de particules dans un solvant siliconé, ledit polymère étant obtenu par polymérisation d'acrylate de méthyle et de macromonomère monométhacryloxypropylpolydiméthylsiloxane (MCR-M17).
Dans un réacteur de 1 litre, on charge 200 g d'heptane, 200 g de phényltrimethicone, 30 g d'acrylate de méthyle, 10 g de macromonomère monométhacryloxypropylpolydiméthylsiloxane (MCR-M17 de Mw = 5000) et 3,2 g de tertiobutylperoxy-2-éthylhexanoate (Trigonox 21S).
On agite et on chauffe le mélange réactionnel à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 160 g d'acrylate de méthyle et 2 g de Trigonox 21S. On laisse ensuite le chauffage pendant 4 heures à 90°C puis on distille l'heptane du milieu réactionnel.
A l'issue de cette opération de distillation, on obtient une dispersion de particules de polymère ainsi préparé stable dans le phényl trimethicone
Les caractéristiques du polymère et des particules formées par ledit polymère sont les suivantes :

- Masse moléculaire poids Mw=95630
- Masse moléculaire nombre Mn=25690
- Indice de polydispersité (Mw/Mn)= 3.72
- Extrait sec théorique : 50 % dans le phényl trimethicone
- Transition vitreuse : 12°C par DSC Mettler
- Granulométrie : 150 nm avec polydispersité de 0,04 réalisée sur Malvern Autosizer Lo-C à 25°C

Le macromonomère représente 5% en poids du polymère.

Après la mise en oeuvre du protocole de stabilité conformément à l'exemple 1, on constate que la dispersion obtenue est stable.

## EXEMPLE 5

Cet exemple illustre la préparation d'un polymère formant une dispersion de particules dans un solvant siliconé, ledit polymère étant obtenu par polymérisation d'acrylate de méthyle, d'acide acrylique et de macromonomère monométhacryloxypropylpolydiméthylsiloxane (MCR-M17).

Dans un réacteur de 1 litre, on charge 200 g d'heptane, 200 g de D5, 26 g d'acrylate de méthyle, 14 g de macromonomère monométhacryloxypropylpolydiméthylsiloxane (MCR-M17 de Mw = 5000) et 3,2 g de tertiobutylperoxy-2-éthylhexanoate (Trigonox 21S).

On agite et on chauffe le mélange réactionnel à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 120 g d'acrylate de méthyle, 40 g d'acide acrylique et 2 g de Trigonox 21S. On laisse ensuite le chauffage pendant 4 heures à 90°C puis on distille l'heptane du milieu réactionnel.

A l'issue de cette opération de distillation, on obtient une dispersion de particules de polymère ainsi préparé stable dans le D5.

Les caractéristiques du polymère et des particules formées par ledit polymère sont les suivantes :

- Extrait sec théorique : 50 % dans le D5, réalisé par thermobalance
- Transition vitreuse : 12°C par DSC Mettler
- Granulométrie : 170 nm avec polydispersité de 0,04 réalisée sur Malvern Autosizer Lo-C à 25°C

Le macromonomère représente 7% en poids du polymère.

Après la mise en oeuvre du protocole de stabilité conformément à l'exemple 1, on constate que la dispersion obtenue est stable.

## Exemple 6 : Composition de mascara

On a préparé un mascara ayant la composition suivante :

| | |
|---|---|
| Cire d'abeille | 8 g |
| Cire de paraffine | 3 g |
| Cire de carnauba | 6 g |
| Hectorite modifiée par du chlorure de di-stéaryl di-méthyl benzyl ammonium (Bentone® 38V d'Elementis) | 5,3 g |
| Carbonate de propylène | 1,7 g |
| Charge | 1 g |
| Pigments | 5 g |
| Dispersion de polymère de l'exemple 1 | 12 g MA* |
| Isododécane | qsp 100 g |

*MA : matière active

Le mascara, après application sur les cils, est jugé très satisfaisant.

## Exemple 7 : Stick de rouge à lèvres

La composition de rouge à lèvres suivante est préparée :

| | |
|---|---|
| Cire de polyéthylène | 15 % |
| Dispersion de polymère de l'exemple 2 | 10 % en MA |
| Polyisobutène hydrogéné (Parléam de Nippon Oil Fats) | 26 % |
| Pigments | 8,6 % |

(suite)

| | |
|---|---|
| Isododécane | qsp 100% |

La composition obtenue après application sur les lèvres présente de bonnes propriétés cosmétiques.

**Exemple 8 : Fond de teint E/H**

On prépare une composition de fond de teint comprenant les composés suivants :

| | | |
|---|---|---|
| Phase A | Cetyl Dimethicone copolyol (ABIL EM 90 de la société GOLDSCHMIDT) | 3 g |
| | Succinate d'isostéaryl diglycéryle (IMWITOR 780K de la société CONDEA) | 0,6 g |
| | Isododécane | 18,5 g |
| | Mélange de pigments (oxydes de fer et oxydes de titane hydrophobes) | 10 g |
| | Dispersion de polymère de l'exemple 3 | 8,7 g en MA |
| | Poudre de polyamide (NYLON-12 de Dupont de Nemours) | 8 g |
| | Parfum | qs |
| Phase B | Eau | qsp 100 g |
| | Sulfate de magnésium | 0,7 g |
| | Conservateur (Methylparaben) | qs |
| Phase C | Eau | 2 g |
| | Conservateur (Diazolinyl urée) | qs |

La composition obtenue présente de bonnes propriétés cosmétiques.

**Exemple 9**

On prépare une poudre compactée ayant la composition suivante :

Composition A :

- Talc 30 g
- Oxychlorure de bismuth 10 g
- Stéarate de zinc 4 g
- Poudre de Nylon 20 g
- Dispersion de l'exemple 1 5 g

Composition B :

- Oxydes de fer 2 g
- Huile de vaseline 6 g

La poudre est obtenue de la façon suivante : on broie la composition A dans un broyeur de type KENWOOD pendant environ 5 minutes sous faible agitation, on ajoute la composition B et on broie l'ensemble environ 2 minutes à la même vitesse, puis 3 minutes à une vitesse plus rapide. On tamise ensuite la préparation sur un tamis de 0,16 mm, puis on compacte ce mélange dans des coupelles.
On obtient une poudre compactée présentant de bonnes propriétés cosmétiques.
La composition obtenue est aisée et agréable à appliquer. On constate que le film ne migre pas dans les ridules de la peau, même après avoir été porté pendant plusieurs heures.

**Exemple 10 : gel pour le visage**

On prépare la composition suivante :

. isopropyl palmitate 10 g
. vaseline (cire) 5 g
. hectorite modifiée (argile) 0,15 g
. ozokérite (cire) 5 g
. septaoléate de sorbitane oxyéthyléné (40OE) 5 g
. dispersion de l'exemple 4 (25% de matière sèche) 75 g

On obtient un gel ayant de bonnes propriétés cosmétiques.

**Exemple 11 : huile de soin**

On prépare la composition suivante :

. dispersion de l'exemple2 (25% de matière sèche) 70 g
. huile de jojoba 15 g
. huile de soja 15g

On obtient une huile de soin qui peut être appliquée sur le corps ou le visage.

## Revendications

**1.** Dispersion, dans un milieu non aqueux siliconé de particules constituées d'au moins un polymère acrylique comprenant un squelette insoluble dans ledit milieu, et une partie soluble dans ledit milieu constituée de chaînes latérales liées de manière covalente audit squelette, ledit polymère étant susceptible d'être obtenu par polymérisation radicalaire dans ledit milieu d'un mélange polymérisable constitué :

- d'un premier monomère choisi parmi les (méth) acrylates d'alkyle en C1- C3, seul ou en mélange, éventuellement en présence d'un ou plusieurs monomères additionnels choisis parmi l'acide acrylique, l'acide méthacrylique et les (méth) acrylates d'alkyle de formule (I), et leurs sels :

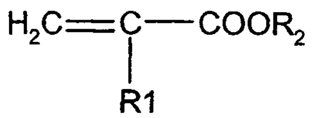

(I)

dans laquelle :

- $R_1$ désigne un atome d'hydrogène ou un groupe méthyle ;
- $R_2$ représente
- un groupe alkyle linéaire ou ramifié comprenant de 1 à 6 atomes de carbone, ledit groupe comportant dans sa chaîne un ou plusieurs atomes d'oxygène, et/ou comportant un ou plusieurs substituants choisis parmi -OH, les atomes d'halogène (F, Cl, Br, I) et -NR'R" avec R' et R" identiques ou différents choisis parmi les alkyles linéaires ou ramifiés en C1-C3;
- un groupe alkyle cyclique comprenant de 3 à 6 atomes de carbone, ledit groupe pouvant comporter dans sa chaîne un ou plusieurs atomes d'oxygène et/ou pouvant comporter un ou plusieurs substituants choisis parmi OH et les atomes d'halogène (F, Cl, Br, I); pour former ledit squelette insoluble; et
- d'au moins un macromonomère siliconé comportant un groupe terminal apte à réagir pendant la polymérisation pour former les chaînes latérales, ledit macromonomère ayant une masse moléculaire en poids supérieure ou égale à 200 et représentant 1 à 18% en poids du polymère.

**2.** Dispersion selon la revendication 1, dans laquelle le milieu non aqueux siliconé est constitué d'au moins 50% en

poids d'au moins un composé liquide non aqueux siliconé ayant un paramètre de solubilité global selon l'espace de solubilité de Hansen inférieur ou égal à 17 $(MPa)^{1/2}$.

**3.** Dispersion selon la revendication 2, dans laquelle le composé non aqueux siliconé est choisi parmi les huiles de silicone, éventuellement ramifiées, volatiles et/ou non volatiles, et les silicones linéaires ou cycliques ayant de 2 à 7 atomes de silicium, ces silicones comportant éventuellement des groupes alkyle ou alkoxy ayant de 1 à 10 atomes de carbone; les polydialkylsiloxanes non volatils, tels que les polydiméthylsiloxanes (PDMS) non volatils; les polydiméthylsiloxanes comportant des groupements alkyle, alcoxy ou phényle, pendant ou en bout de chaîne siliconée, groupements ayant de 2 à 24 atomes de carbone; les silicones phénylées comme les phényl triméthicones, les phényl diméthicones, les phényl triméthylsiloxy diphénylsiloxanes, les diphényl diméthicones, les diphényl méthyldiphényl trisiloxanes, les polyméthylphénylsiloxanes; les polysiloxanes modifiés par des acides gras (notamment en C8-C20), des alcools gras (notamment en C8-C20) ou des polyoxyalkylènes (notamment polyoxyéthyléne et/ou polyoxypropylène); les polysiloxanes aminées; les polysiloxanes à groupement hydroxyles; les polysiloxanes fluorés comportant un groupement fluoré pendant ou en bout de chaîne siliconée ayant de 1 à 12 atomes de carbone dont tout ou partie des hydrogène sont substitués par des atomes de fluor; et leurs mélanges.

**4.** Dispersion selon l'une des revendications précédentes, dans laquelle le premier monomère, ou mélange de premiers monomères, représente 50-100% en poids, notamment 55 à 95% en poids, voire 60 à 80% en poids du mélange "premier(s) monomère(s) + monomère(s) additionnel(s)".

**5.** Dispersion selon l'une des revendications précédentes, dans laquelle ledit premier monomère est choisi parmi, seul ou en mélange, l'acrylate de méthyle, le méthacrylate de méthyle, l'acrylate d'éthyle, le méthacrylate d'éthyle, l'acrylate de n-propyle, le méthacrylate de n-propyle, l'acrylate d'isopropyle et le méthacrylate d'isopropyle.

**6.** Dispersion selon l'une des revendications précédentes, dans laquelle le monomère additionnel est choisi parmi, seul ou en mélange, les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle et leurs sels.

**7.** Dispersion selon l'une des revendications précédentes, dans laquelle le macromonomère constituant après réaction les chaînes latérales du polymère selon l'invention, comporte en bout de chaîne un groupe terminal polymérisable choisi parmi un groupe vinyle ou (méth)acryloyloxy (acrylate ou méthacrylate).

**8.** Dispersion selon l'une des revendications précédentes, dans laquelle le macromonomère présente une masse moléculaire en poids (Mw) allant de 200 à 100 000, de préférence de 300 à 50 000, notamment 500 à 20 000, préférentiellement de 800 à 10000, voire de 1000 à 6000.

**9.** Dispersion selon l'une des revendications précédentes, dans laquelle le macromonomère est choisi parmi les polydiméthylsiloxanes à groupement terminal monoacryloxy ou monométhacryloxy, et notamment ceux de formule (II) suivante :

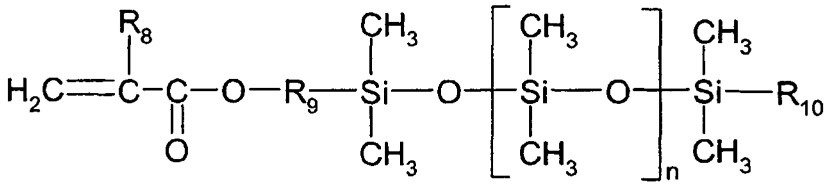

(II)

dans laquelle :

- $R_8$ désigne un atome d'hydrogène ou un groupement méthyle ; de préférence méthyle;
- $R_9$ désigne un groupe hydrocarboné divalent ayant de 1 à 10 atomes de carbone et contenant éventuellement une ou deux liaisons éther -O- ; de préférence éthylène, propylène ou butylène;
- $R_{10}$ désigne un groupe alkyle ayant de 1 à 10 atomes de carbone, notamment de 2 à 8 atomes de carbone ; de préférence méthyle, éthyle, propyle, butyle ou pentyle;
- n désigne un nombre entier allant de 1 à 300, de préférence allant de 3 à 200, et préférentiellement allant de 5 à 100.

**10.** Dispersion selon l'une des revendications précédentes, dans laquelle le macromonomère est présent dans le polymère dans une proportion de 2 à 16% en poids, préférentiellement de 4 à 15% en poids, et notamment de 6 à 12% en poids, voire 8 à 10% en poids, par rapport au poids total dudit polymère.

**11.** Dispersion selon l'une des revendications précédentes, dans laquelle la masse moléculaire en poids (Mw) du polymère est comprise entre 10 000 et 300 000, notamment entre 20 000 et 200 000, mieux encore entre 25 000 et 150 000.

**12.** Dispersion selon l'une des revendications précédentes, dans laquelle les particules de polymères sont des particules solides de taille moyenne allant de 10 à 400 nm, de préférence de 20 à 200 nm.

**13.** Dispersion selon l'une des revendications précédentes, présentant un taux de matière sèche (ou extrait sec) de 40% à 70% en poids de matière sèche, notamment de 45 à 65% en poids.

**14.** Composition cosmétique ou pharmaceutique comprenant, dans un milieu cosmétiquement ou pharmaceutiquement acceptable, une dispersion selon l'une des revendications précédentes.

**15.** Composition selon la revendication 14, dans laquelle la dispersion est présente à raison de 3 à 95% en poids dans la composition, notamment 4-90% en poids, voire 20-70% en poids.

**16.** Composition selon l'une des revendications 14 à 15, comprenant, en outre, des corps gras choisis parmi les cires, les huiles, les gommes, les corps gras pâteux, hydrocarbonés ou siliconés; et/ou des composés pulvérulents tels que les pigments, les charges et/ou les nacres; et/ou des antioxydants, des parfums, des huiles essentielles, des conservateurs, des actifs cosmétiques, des hydratants, des vitamines, des acides gras essentiels, des sphingolipides, des filtres solaires, des tensioactifs, des polymères liposolubles comme les polyalkylènes, notamment le polybutène, les polyacrylates et les polymères siliconés compatibles avec les corps gras.

**17.** Composition selon l'une des revendications 14 à 16, se présentant sous la forme d'une composition de soin, de nettoyage ou de maquillage pour la peau ou les matières kératiniques, d'une composition capillaire, d'une composition solaire.

**18.** Procédé de traitement cosmétique pour le soin, le nettoyage et/ou le maquillage des matières kératiniques telles que la peau, le cuir chevelu, les cils, les sourcils, les lèvres, les ongles, consistant à appliquer sur lesdites matières kératiniques, une composition telle que définie à l'une des revendications 14 à 17.

**Claims**

**1.** Dispersion, in a non-aqueous, silicone medium, of particles consisting of at least one acrylic polymer comprising a skeleton that is insoluble in the said medium, and a portion that is soluble in the said medium, consisting of side chains covalently bonded to the said skeleton, the said polymer being able to be obtained by free-radical polymerization in the said medium of a polymerizable mixture consisting of:

- a first monomer chosen from C1- C3 alkyl (meth) acrylates, alone or as a mixture, optionally in the presence of one or more additional monomers chosen from acrylic acid, methacrylic acid and alkyl (meth) acrylates of formula (I), and the salts thereof,

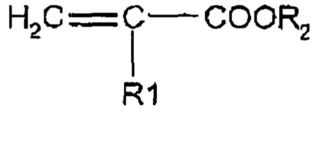

(I)

in which:

- $R_1$ denotes a hydrogen atom or a methyl group;
- $R_2$ represents:

- a linear or branched alkyl group containing from 1 to 6 carbon atoms, the said group containing in its chain one or more oxygen atoms and/or containing one or more substituents chosen from -OH, halogen atoms (F, Cl, Br or I) and -NR' R" with R' and R", which may be identical or different, chosen from linear or branched C1-C3 alkyls;
- a cyclic alkyl group containing from 3 to 6 carbon atoms, the said group possibly comprising in its chain one or more oxygen atoms and/or possibly comprising one or more substituents chosen from OH and halogen atoms (F, Cl, Br or I); to form the said insoluble skeleton; and

- of at least one silicone macromonomer comprising an end group capable of reacting during the polymerization to form the side chains, the said macromonomer having a weight-average molecular mass of greater than or equal to 200 and representing 1% to 18% by weight of the polymer.

**2.** Dispersion according to Claim 1, in which the non-aqueous silicone medium consists of at least 50% by weight of at least one non-aqueous silicone liquid compound having a global solubility parameter according to the Hansen solubility space of less than or equal to 17 $(MPa)^{1/2}$.

**3.** Dispersion according to Claim 2, in which the non-aqueous silicone compound is chosen from optionally branched, volatile and/or non-volatile silicone oils, and linear or cyclic silicones containing from 2 to 7 silicon atoms, these silicones optionally comprising alkyl or alkoxy groups containing from 1 to 10 carbon atoms; non-volatile polydialkylsiloxanes, such as non-volatile polydimethylsiloxanes (PDMSs); polydimethylsiloxanes comprising alkyl, alkoxy or phenyl groups, which are pendent or at the end of a silicone chain, these groups containing from 2 to 24 carbon atoms; phenylsilicones, for instance phenyl trimethicones, phenyl dimethicones, phenyltrimethylsiloxydiphenylsiloxanes, diphenyl dimethicones, diphenylmethyldiphenyltrisiloxanes and polymethylphenylsiloxanes; polysiloxanes modified with fatty acids (especially of $C_8$-$C_{20}$), fatty alcohols (especially of $C_8$-$C_{20}$) or polyoxyalkylenes (especially polyoxyethylene and/or polyoxypropylene); amino polysiloxanes; polysiloxanes containing hydroxyl groups; fluoro polysiloxanes comprising a fluoro group that is pendent or at the end of a silicone chain, containing from 1 to 12 carbon atoms, all or some of the hydrogens of which are replaced with fluorine atoms; and mixtures thereof.

**4.** Dispersion according to one of the preceding claims, in which the first monomer, or mixture of first monomers, represents 50-100% by weight, especially 55 to 95% by weight or even 60 to 80% by weight of the mixture "first monomer(s) + additional monomer(s)".

**5.** Dispersion according to one of the preceding claims, in which the said first monomer is chosen, alone or as a mixture, from methyl acrylate, methyl methacrylate, ethyl acrylate, ethyl methacrylate, n-propyl acrylate, n-propyl methacrylate, isopropyl acrylate and isopropyl methacrylate.

**6.** Dispersion according to one of the preceding claims, in which the additional monomer is chosen, alone or as a mixture, from methoxyethyl or ethoxyethyl (meth)acrylates; trifluoroethyl methacrylate; dimethylaminoethyl methacrylate, diethylaminoethyl methacrylate, 2-hydroxypropyl methacrylate, 2-hydroxyethyl methacrylate, 2-hydroxypropyl acrylate, 2-hydroxyethyl acrylate; and the salts thereof.

**7.** Dispersion according to one of the preceding claims, in which the macromonomer constituting, after reaction, the side chains of the polymer according to the invention comprises at the end of the chain a polymerizable end group chosen from a vinyl group and a (meth)acryloxy group (acrylate or methacrylate).

**8.** Dispersion according to one of the preceding claims, in which the macromonomer has a weight-average molecular mass (Mw) ranging from 200 to 100 000, preferably from 300 to 50 000, especially 500 to 20 000, preferentially from 800 to 10 000 and even from 1000 to 6000.

**9.** Dispersion according to one of the preceding claims, in which the macromonomer is chosen from polydimethylsiloxanes containing a monoacryloxy or monomethacryloxy end group, and especially those of formula (II) below:

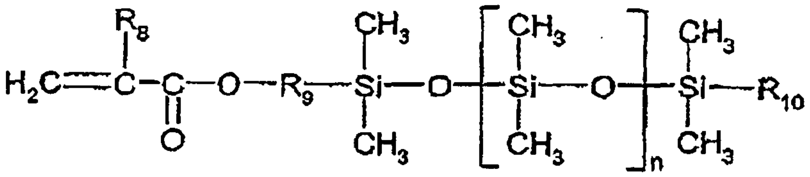 (II)

in which:

- $R_8$ denotes a hydrogen atom or a methyl group; preferably methyl;
- $R_9$ denotes a divalent hydrocarbon-based group containing from 1 to 10 carbon atoms and optionally containing one or two ether bonds -O-; preferably ethylene, propylene or butylene;
- $R_{10}$ denotes an alkyl group containing from 1 to 10 carbon atoms and especially from 2 to 8 carbon atoms; preferably methyl, ethyl, propyl, butyl or pentyl;
- n denotes an integer ranging from 1 to 300, preferably ranging from 3 to 200 and preferentially ranging from 5 to 100.

**10.** Dispersion according to one of the preceding claims, in which the macromonomer is present in the polymer in a proportion of from 2% to 16% by weight, preferably from 4% to 15% by weight and especially from 6% to 12% by weight or even from 8% to 10% by weight relative to the total weight of the said polymer.

**11.** Dispersion according to one of the preceding claims, in which the weight-average molecular mass (Mw) of the polymer is between 10 000 and 300 000, especially between 20 000 and 200 000 and better still between 25 000 and 150 000.

**12.** Dispersion according to one of the preceding claims, in which the polymer particles are solid particles having a mean size ranging from 10 to 400 nm and preferably from 20 to 200 nm.

**13.** Dispersion according to one of the preceding claims, with a solids content (or dry extract) of from 40% to 70% by weight of solids and especially from 45% to 65% by weight.

**14.** Cosmetic or pharmaceutical composition comprising, in a cosmetically or pharmaceutically acceptable medium, a dispersion according to one of the preceding claims.

**15.** Composition according to Claim 14, in which the dispersion is present in a proportion of from 3% to 95% by weight in the composition, especially 4-90% by weight or even 20-70% by weight.

**16.** Composition according to either of Claims 14 and 15, also comprising fatty substances chosen from waxes, oils, gums and pasty fatty substances, which are hydrocarbon-based or silicone-based; and/or pulverulent compounds such as pigments, fillers and/or nacres; and/or antioxidants, fragrances, essential oils, preserving agents, cosmetic active agents, moisturizers, vitamins, essential fatty acids, sphingolipids, sunscreens, surfactants, liposoluble polymers, for instance polyalkylenes, especially polybutene, polyacrylates and silicone polymers that are compatible with fatty substances.

**17.** Composition according to one of Claims 14 to 16, which is in the form of a care, cleansing or makeup composition for the skin or keratin materials, a haircare composition or an antisun composition.

**18.** Cosmetic treatment process for caring for, cleansing and/or making up keratin materials such as the skin, the scalp, the eyelashes, the eyebrows, the lips and the nails, which consists in applying a composition as defined in one of Claims 14 to 17 to the said keratin materials.

## Patentansprüche

**1.** Dispersion, in einem nichtwässrigen Siliconmedium, von Partikeln, die aus mindestens einem Acrylpolymer bestehen, das ein in dem Medium unlösliches Rückgrat und einen in dem Medium löslichen Teil aufweist, der aus seitenständigen Ketten besteht, die kovalent mit dem Rückgrat verbunden sind, wobei das Polymer durch radikalische

Polymerisation eines polymerisierbaren Gemischs in dem Medium erhalten werden kann, das besteht aus:

- einem ersten Monomer, das unter den $C_{1-3}$-Alkyl (meth)-acrylaten, einzeln oder im Gemisch, ausgewählt wird, gegebenenfalls in Gegenwart eines oder mehrerer zusätzlicher Monomere, die unter Acrylsäure, Methacrylsäure und den Alkyl (meth) acrylaten der Formel (I):

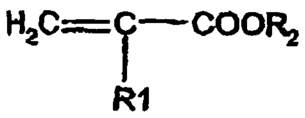

(I)

und ihren Salzen ausgewählt werden, in der:

- $R_1$ ein Wasserstoffatom oder eine Methylgruppe bedeutet;
- $R_2$

  - eine geradkettige oder verzweigte Alkylgruppe, die 1 bis 6 Kohlenstoffatome aufweist, wobei die Gruppe in ihrer Kette ein oder mehrere Sauerstoffatome enthält und/oder einen oder mehrere Substituenten aufweist, die ausgewählt werden unter -OH, den Halogenatomen (F, Cl, Br, I) und -NR'R", worin die Reste R' und R" identisch oder verschieden sind und unter den geradkettigen oder verzweigten $C_{1-3}$-Alkylen ausgewählt werden;
  - eine cyclische Alkylgruppe, die 3 bis 6 Kohlenstoffatome aufweist, wobei die Gruppe in ihrer Kette ein oder mehrere Sauerstoffatome enthalten kann und/oder einen oder mehrere Substituenten enthalten kann, die unter OH und den Halogenatomen (F, Cl, Br, I) ausgewählt werden, enthalten kann;

darstellt,
für die Erzeugung des unlöslichen Rückgrats; und
- mindestens einen Siliconmakromonomer, das eine endständige Gruppe aufweist, die imstande ist, während der Polymerisation unter Bildung der seitenständigen Ketten zu reagieren, wobei das Makromonomer eine gewichtsbezogene Molekularmasse von 200 oder darüber aufweist und 1 bis 18 Gew.-% des Polymers ausmacht.

**2.** Dispersion nach Anspruch 1, wobei das nichtwässrige Siliconmedium zu mindestens 50 Gew.-% aus mindestens einer nichtwässrigen flüssigen Siliconverbindung besteht, die ein Gesamtlöslichkeitsparameter gemäß dem Hansen-Löslichkeitsraum von 17 $(MPa)^{1/2}$ oder darunter aufweist.

**3.** Dispersion nach Anspruch 2, wobei die nichtwässrige Siliconverbindung unter den flüchtigen und/oder nichtflüchtigen, gegebenenfalls verzweigten Siliconölen, und den geradkettigen oder cyclischen Siliconen, die 2 bis 7 Siliciumatome aufweisen, wobei diese Silicone gegebenenfalls Alkyl- oder Alkoxygruppen aufweisen, die 1 bis 10 Kohlenstoffatome enthalten; den nichtflüchtigen Polydialkylsiloxanen, wie den nichtflüchtigen Polydimethylsiloxanen (PDMS); den Polydimethylsiloxanen, die Alkyl-, Alkoxy- oder Phenylgruppen aufweisen, seitenständig oder am Ende der Siliconkette, wobei die Gruppen 2 bis 24 Kohlenstoffatome aufweisen; den phenylierten Siliconen, wie den Phenyltrimethiconen, den Phenyldimethiconen, den Phenyltrimethylsiloxydiphenylsiloxanen, den Diphenyldimethiconen, den Diphenylmethyldiphenyltrisiloxanen, den Polymethylphenylsiloxanen; den Polysiloxanen, die mit Fettsäuren (insbesondere $C_{8-20}$), Fettalkoholen (insbesondere $C_{8-20}$) oder Polyoxyalkylenen (insbesondere Polyoxyethylen und/oder Polyoxypropylen) modifiziert sind; den aminierten Polysiloxanen, den Polysiloxanen mit Hydroxygruppe(n); den fluorierten Polysiloxanen, die eine seitenständige oder am Ende der Siliconkette angeordnete fluorierte Gruppe aufweisen, die 1 bis 12 Kohlenstoffatome enthält, bei der der gesamte Wasserstoff oder ein Teil des Wasserstoffs durch Fluoratome substituiert ist, und deren Gemischen ausgewählt wird.

**4.** Dispersion nach einem der vorhergehenden Ansprüche, wobei das erste Monomer oder das Gemisch erster Monomere 50-100 Gew.-%, insbesondere 55 bis 95 Gew.-%, sogar 60 bis 80 Gew.-% des Gemischs "erstes Monomer/erste Monomere + zusätzliches Monomer/zusätzliche Monomere" ausmacht.

**5.** Dispersion nach einem der vorhergehenden Ansprüche, wobei das erste Monomer, einzeln oder im Gemisch, unter Methylacrylat, Methylmethacrylat, Ethylacrylat, Ethylmethacrylat, *n*-Propylacrylat, *n*-Propylmethacrylat, Isopropylacrylat und Isopropylmethacrylat ausgewählt wird.

**6.** Dispersion nach einem der vorhergehenden Ansprüche, wobei das zusätzliche Monomer, einzeln oder im Gemisch, unter Methoxyethyl- oder Ethoxyethyl(meth)acrylat, Trifluorethylmethacrylat, Dimethylaminoethylmethacrylat, Diethylaminoethylmethacrylat, 2-Hydroxypropylmethacrylat, 2-Hydroxyethylmethacrylat, 2-Hydroxypropylacrylat, 2-Hydroxyethylacrylat und deren Salzen ausgewählt wird.

**7.** Dispersion nach einem der vorhergehenden Ansprüche, wobei das Makromonomer, das nach der Umsetzung die seitenständigen Ketten des erfindungsgemäßen Polymers bildet, am Kettenende eine endständige polymerisierbare Gruppe aufweist, die unter Vinyl oder (Meth)acryloyloxyacrylat oder (Meth)acryloyloxymethacrylat ausgewählt wird.

**8.** Dispersion nach einem der vorhergehenden Ansprüche, wobei das Makromonomer eine gewichtsbezogene Molekularmasse (Mw) aufweist, die im Bereich von 200 bis 100 000, vorzugsweise 300 bis 50 000, insbesondere 500 bis 20 000, vorzugsweise 800 bis 10 000, sogar 1 000 bis 6 000 liegt.

**9.** Dispersion nach einem der vorhergehenden Ansprüche, wobei das Makromonomer unter den Polydimethylsiloxanen mit endständiger Monoacryloxy- oder Monomethacryloxygruppe und insbesondere unter den Polydimethylsiloxanen der folgenden Formel (II):

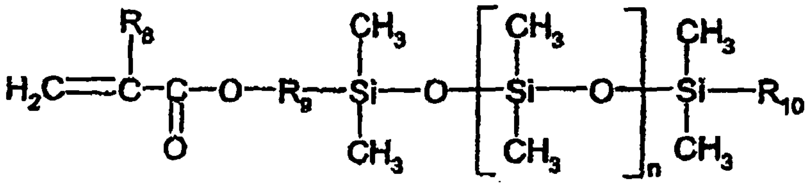
(II)

ausgewählt wird, in der bedeuten:

- $R_8$ ein Wasserstoffatom oder eine Methylgruppe; vorzugsweise Methyl;
- $R_9$ eine zweiwertige Kohlenwasserstoffgruppe, die 1 bis 10 Kohlenstoffatome aufweist und gegebenenfalls eine oder zwei Etherbindungen -O- enthält; und vorzugsweise Ethylen, Propylen oder Butylen;
- $R_{10}$ eine Alkylgruppe, die 1 bis 10 Kohlenstoffatome, insbesondere 2 bis 8 Kohlenstoffatome, aufweist; vorzugsweise Methyl, Ethyl, Propyl, Butyl oder Pentyl;
- n eine ganze Zahl, die im Bereich von 1 bis 300, vorzugsweise 3 bis 200 und vorzugsweise 5 bis 100 liegt.

**10.** Dispersion nach einem der vorhergehenden Ansprüche, wobei das Makromonomer in dem Polymer in einem Anteil von 2 bis 16 Gew.-%, vorzugsweise 4 bis 15 Gew.-% und insbesondere 6 bis 12 Gew.-%, sogar 8 bis 10 Gew.-%, bezogen auf das Gesamtgewicht des Polymers, vorhanden ist.

**11.** Dispersion nach einem der vorhergehenden Ansprüche, wobei die gewichtsbezogene Molekularmasse (Mw) des Polymers im Bereich von 10 000 bis 300 000, insbesondere 20 000 bis 200 000, noch besser 25 000 bis 150 000 liegt.

**12.** Dispersion nach einem der vorhergehenden Ansprüche, wobei die Polymerpartikel feste Partikel mit einer mittleren Größe sind, die im Bereich von 10 bis 400 nm, vorzugsweise 20 bis 200 nm liegt.

**13.** Dispersion nach einem der vorhergehenden Ansprüche, die einen Trockensubstanzgehalt (oder Trockenextraktgehalt) von 40 bis 70 Gew.-% Trockensubstanz, insbesondere 45 bis 65 Gew.-% aufweist.

**14.** Kosmetische oder pharmazeutische Zusammensetzung, die in einem kosmetisch oder pharmazeutisch akzeptablen Medium eine Dispersion nach einem der vorhergehenden Ansprüche enthält.

**15.** Zusammensetzung nach Anspruch 14, wobei die Dispersion in einem Anteil von 3 bis 95 Gew.-%, insbesondere 4 bis 90 Gew.-%, sogar 20 bis 70 Gew.-%, in der Zusammensetzung vorhanden ist.

**16.** Zusammensetzung nach einem der Ansprüche 14 bis 15, die außerdem enthält; Fettsubstanzen, die unter Wachsen, Ölen, Gummen, pastösen Fettsubstanzen, die kohlenwasserstoffhaltig oder siliconiert sind, ausgewählt werden;

und/oder pulverförmige Verbindungen, wie Pigmente, Füllstoffe und/oder Perlglanzpigmente; und/oder Antioxidantien, Parfüms, etherische Öle, Konservierungsmittel, kosmetische Wirkstoffe, Hydratisierungsmittel, Vitamine, essentielle Fettsäuren, Sphingolipide, Sonnenschutzfilter, grenzflächenaktive Stoffe, fettlösliche Polymere, wie die Polyalkylene, insbesondere Polybutylen, die Polyacrylate und die Siliconpolymere, die mit den Fettsubstanzen kompatibel sind.

**17.** Zusammensetzung nach einem der Ansprüche 14 bis 16, die in Form einer Zusammensetzung zum Pflegen, Reinigen oder Schminken für die Haut oder die Keratinmaterialien, einer Haarbehandlungszusammensetzung, einer Sonnenschutzzusammensetzung vorliegt.

**18.** Kosmetisches Behandlungsverfahren zum Pflegen, Reinigen und/oder Schminken von Keratinmaterialien, wie der Haut, der Kopfhaut, der Wimpern, der Augenbrauen, der Lippen, der Nägel, das darin besteht, auf die Keratinmaterialien eine wie in einem der Ansprüche 14 bis 17 definierte Zusammensetzung aufzutragen.